# EUROPEAN PATENT APPLICATION

(11) **EP 4 406 494 A1**
(43) Date of publication of application: **31.07.2024**
(21) Application number: 22871621.3
(22) Date of filing: 29.07.2022
(51) Int. Cl.: A61B 17/072

(54) **TRAVEL DETECTION STRUCTURE AND CONTROL METHOD THEREFOR, ELECTRIC STAPLER, AND MEDICAL DEVICE**

(30) Priority: 26.09.2021 CN 202111146936
(71) Applicant: Surgnova Healthcare Technologies (Zhejiang) Co., Ltd., Ningbo, Zhejiang 315300 (CN)
(72) Inventor: LI, Guangrong, Ningbo, Zhejiang 315300 (CN); LI, Yang, Ningbo, Zhejiang 315300 (CN); ZHANG, Yaning, Ningbo, Zhejiang 315300 (CN); LI, ZONGLEI, Ningbo, Zhejiang 315300 (CN)
(74) Representative: Algemeen Octrooi- en Merkenbureau B.V.
(86) International application number: PCT/CN2022/109119
(87) International publication number: WO 2023/045563

(57) **Abstract**

A travel detector and a control method thereof, an electric stapler, and a medical device. The travel detector is applied to a detection on a firing action of the medical device. The travel detector includes: a supporting portion, serving as a supporting structure of the travel detector; a transmission portion penetrating into the supporting portion and arranged to be movable in an axial direction in the supporting portion; and a detecting portion fixed on the supporting portion and in contact with the transmission portion through the supporting portion. An end of the transmission portion is connected to an operating end of the medical device. When the transmission portion moves in the axial direction in the supporting portion, the transmission portion sequentially comes into contact with different detecting positions on the detecting portion to complete a travel detection of the transmission portion relative to the supporting portion, so that the operating end performs the firing action according to the travel detection. It is possible to completely avoid the problem of excessive squeezing through the travel detection, and achieve a detection of different detecting positions, such as a detection with a staple cartridge secondary firing lock.

## Description

### TECHNICAL FIELD

The present disclosure relates to a field of medical device technology, and in particular to, a travel detector, a method of controlling a travel detector, an electric stapler, and a medical device.

### BACKGROUND

At present, electric staplers in a related art are mainly divided into two types: semi-automatic electric staplers and fully automatic electric staplers. The problems that the existing electric stapler encounters in a specific operation process include: (1) as the target tissue needs to be squeezed before anastomosis and cutting by the stapler, when an anvil of the electric stapler is closed to squeeze the target tissue, there may be excessive squeezing force from the anvil, which will over-squeeze the target tissue that is too thick, and a low height staple cartridge is used to be anastomosis with the thick tissue, so that there may be staple bursts, resulting in failed anastomosis and the target tissue being prone to significant bleeding; (2) the staple cartridge of the stapler is a disposable device that needs to be replaced multiple times during the surgical process, and if reused, it may result in tissue being only cut without anastomosis, causing organ damage, bleeding, and serious surgical accidents in patients.

### SUMMARY

An aspect of the present disclosure provides a travel detector, applied to a detection on a firing action of a medical device, including a supporting portion, a transmission portion, and a detecting portion. The supporting portion serves as a supporting structure of the travel detector. The transmission portion penetrates into the supporting portion and is arranged to be movable in an axial direction in a supporting body. The detecting portion is fixed on the supporting portion and in contact with the transmission portion through the supporting portion. An end of the transmission portion is connected to an operating end of the medical device. When the transmission portion moves in the axial direction in the supporting portion, the transmission portion sequentially comes into contact with different detecting positions on the detecting portion to complete a travel detection of the transmission portion relative to the supporting portion, so that the operating end performs the firing action according to the travel detection.

According to an embodiment of the present disclosure, the supporting portion includes a supporting frame and a fixed plate. The supporting frame is a frame structure with a central space, and is provided with a plurality of different disposing positions for disposing the transmission portion and the detecting portion, respectively. The fixed plate corresponds to one of the plurality of different disposing positions, and is recessed on a side of the supporting frame to form a disposing space for the detecting portion. The fixed plate includes a plurality of first fixed holes for fixing the detecting portion. The fixed plate is integrally formed with the supporting frame.

According to an embodiment of the present disclosure, the detecting portion includes a plurality of thimbles and a thimble plate. The plurality of thimbles correspond to the plurality of first fixed holes one by one. A hard sheath of each of the plurality of thimbles penetrates through a corresponding first fixed hole. A head of the thimble is facing towards an inside of the supporting frame. The thimble plate is matched with the fixed plate and is disposed in the disposing space. A limiting seat of each of the plurality of thimbles is disposed between the thimble plate and the fixed plate.

According to an embodiment of the present disclosure, the thimble plate includes a plurality of second fixed holes. The plurality of thimbles correspond to the plurality of second fixed holes one by one. A fixed head of each of the plurality of thimbles penetrates through a corresponding second fixed hole. The fixed head is facing towards an outside of the supporting frame.

According to an embodiment of the present disclosure, the plurality of thimbles include a first set of thimbles and a second set of thimbles. The first set of thimbles is disposed corresponding to a set of first fixed holes on the fixed plate. The second set of thimbles is disposed corresponding to a set of second fixed holes on the fixed plate. A setting spacing is between the second set of thimbles and the first set of thimbles.

According to an embodiment of the present disclosure, the first set of thimbles and the second set of thimbles are staggered in the axial direction. A spacing between any two adjacent thimbles in the axial direction is equal. The first set of thimbles and the second set of thimbles each includes at least one thimble connected to a corresponding detecting common end.

According to an embodiment of the present disclosure, the transmission portion includes a transmission strip and a circuit board. The transmission strip is a long rod-shaped structure. An end of the transmission strip is connected to the operating end. When the transmission strip moves relative to the central space of the supporting frame in the axial direction, the transmission strip transmits an action of a forward and backward movement to the operating end. The circuit board is a long plate-shaped structure disposed on a side surface of the transmission strip. When the transmission strip moves relatively in the axial direction, the circuit board comes into different contacts with the plurality of thimbles to determine different travels of the transmission strip, so that the operating end completes the travel detection of the firing action.

According to an embodiment of the present disclosure, the circuit board includes a plurality of contacts. The plurality of contacts include a first set of contacts and a second set of contacts. The first set of contacts is in corresponding contact with the first set of thimbles, and is configured to achieve a retraction zero position detection and a secondary firing lock position detection. The second set of contacts is in corresponding contact with the second set of thimbles, and is configured to achieve a closed zero position detection and a travel endpoint position detection.

According to an embodiment of the present disclosure, the first set of contacts includes a first sub-set of contacts and a second sub-set of contacts. The first sub-set of contacts includes two contacts provided at a top of the circuit board. The two contacts are respectively in contact with two adjacent thimbles in the first set of thimbles to achieve the retraction zero position detection. The second sub-set of contacts includes two contacts provided adjacent to the first sub-set of contacts. The two contacts are respectively in contact with first and last thimbles in the first set of thimbles to achieve the secondary firing lock position detection.

According to an embodiment of the present disclosure, the second set of contacts includes a third sub-set of contacts and a fourth sub-set of contacts. The third sub-set of contacts includes two contacts provided on a lower side of the first sub-set of contacts. The two contacts are respectively in contact with two adjacent thimbles in the second set of thimbles to achieve the closed zero position detection. The fourth sub-set of contacts includes two contacts provided at an end of the circuit board. The two contacts are respectively in contact with first and last thimbles in the second set of thimbles to achieve the travel endpoint position detection.

According to an embodiment of the present disclosure, a first spacing between the two contacts in the first sub-set of contacts in the axial direction is equal to a third spacing between the two contacts in the third sub-set of contacts in the axial direction. A second spacing between the two contacts in the second sub-set of contacts in the axial direction is equal to a fourth spacing between the two contacts in the fourth sub-set of contacts in the axial direction. A width of the two contacts in the first sub-set of contacts in the axial direction is greater than a width of the two contacts in the second sub-set of contacts in the axial direction. The width of the two contacts in the second sub-set of contacts in the axial direction is equal to a width of the two contacts in the third sub-set of contacts in the axial direction. The width of the two contacts in the second sub-set of contacts in the axial direction is equal to a width of the two contacts of the fourth sub-set of contacts in the axial direction.

Another aspect of the present disclosure provides a method of controlling the travel detector as described above, applied to a detection on a firing action of a medical device, including: detecting that the transmission portion of the travel detector is located at the retraction zero position, in response to a detection instruction; controlling the transmission portion to move relative to the supporting portion of the travel detector in the axial direction, in response to the retraction zero position; and controlling the transmission portion to contact with different detecting positions on the detecting portion to complete the travel detection of the transmission portion relative to the supporting portion, so that the operating end performs the firing action according to the travel detection.

Another aspect of the present disclosure provides an electric stapler, including the travel detector as described above and a jaw component. The travel detector is configured to detect the travel of the transmission portion of the travel detector relative to the supporting portion of the travel detector. The jaw component is connected to the supporting portion of the travel detector and serves as the operating end to perform the firing action according to the travel detection.

Another aspect of the present disclosure provides a medical device, including the travel detector as described above.

### BRIEF DESCRIPTION OF DRAWINGS

FIG. 1 schematically shows a three-dimensional composition diagram of a travel detector from a perspective according to an embodiment of the present disclosure;
FIG. 2 schematically shows a local three-dimensional composition diagram of a travel detector from another perspective of a travel detector according to an embodiment of the present disclosure;
FIG. 3 schematically shows a three-dimensional explosion diagram of a travel detector from a perspective according to an embodiment of the present disclosure;
FIG. 4 schematically shows a three-dimensional explosion diagram of a travel detector corresponding to the transmission portion and the detecting portion in FIG. 3 according to an embodiment of the present disclosure;
FIG. 5 schematically shows a three-dimensional composition diagram of a transmission portion of the travel detector in contact with a detecting portion of the travel detector according to an embodiment of the present disclosure;
FIG. 6 schematically shows a plan distribution diagram of contacts of a circuit board of a transmission portion of a travel detector according to an embodiment of the present disclosure;
FIG. 7 schematically shows a plan composition diagram of a thimble of a detecting portion of a travel detector according to an embodiment of the present disclosure; and
FIG. 8 schematically shows a flowchart of a method of controlling a travel detector according to an embodiment of the present disclosure.

### DETAILED DESCRIPTION OF EMBODIMENTS

In order to make the purposes, technical solutions, and advantages of the present disclosure clearer, the present disclosure is further explained in detail combining specific embodiments and referring to accompanying drawings.

It should be noted that the implementation methods not shown or described in the accompanying drawings or the specification are all in forms known to those ordinary skilled in the art, which are not explained in detail. In addition, the above definitions of each element and method are not limited to the various specific structures, shapes, or methods described in embodiments, which may be simply modified or replaced by those ordinary skilled in the art.

It should also be noted that the directional terms described in embodiments, such as "up", "down", "front", "back", "left", "right", etc., are only for reference to the direction of the drawings and are not intended to limit the scope of the protection of the present disclosure. Throughout the drawings, the same elements are represented by the same or similar reference numbers. Existing structures or constructions will be omitted when they may cause confusion in the understanding of the present disclosure.

In addition, the shape and size of each component in the drawings do not reflect the real size and proportion, but only represent the content of embodiments of the present disclosure. Furthermore, in the claims, any reference symbol located between parentheses should not be constructed as a limitation on the claims.

Furthermore, the word "including" does not exclude the presence of elements or steps not listed in the claims. The word "one" or "a" before an element does not exclude the existence of a plurality of such elements.

The use of ordinal numbers such as "first", "second", "third", etc. in the specification and claims for describing the corresponding elements does not imply that the element has any ordinal numbers, nor does it represent the order of one element and another element or the order of the manufacturing method. The use of these ordinal numbers is only to enable an element with a certain name to be clearly distinguished from another element with the same name.

Those skilled in the art may understand that the modules in the devices in embodiments may be adaptively changed and provided in one or more devices different from embodiments. The modules, units, or components in embodiments may be combined into one module, unit, or component. In addition, the modules, units, or components in embodiments may be divided into a plurality of sub-modules, sub-units, or sub-components. Except for at least some of such features and/or processes or units that are mutually exclusive, any combination may be used to combine all features disclosed in the specification (including accompanying claims, abstracts, and drawings), as well as all processes or units of any method or device thus disclosed. Unless otherwise explicitly stated, each feature disclosed in the specification (including accompanying claims, abstracts, and drawings) may be replaced by alternative features that provide the same, equivalent, or similar purposes. Moreover, in the unit claims listing several devices, several of these devices may be specifically embodied through the same hardware item.

Similarly, it should be understood that in order to streamline the present disclosure and assist in understanding one or more aspects of the present disclosure, in the description of exemplary embodiments of the present disclosure above, the various features of the present disclosure are sometimes grouped together into a single embodiment, figure, or description thereof. However, the disclosed method should not be interpreted as reflecting the intention that the claimed protection of the present disclosure has more features than those explicitly recorded in each claim. More precisely, as reflected in the following claims, the disclosure aspects are less than all features of the previously disclosed single embodiment. Therefore, the claims that follow the specific implementation method are explicitly incorporated into the specific implementation method, where each claim itself serves as a separate embodiment of the present disclosure.

At present, electric staplers in circulation on the market are divided into two types: semi-automatic electric staplers and fully automatic electric staplers. Electric staplers mainly include a staple cartridge, a jaw, a firing mechanism, a deflection mechanism, a position switch or a sensor, a retraction mechanism, a main control circuit, and a battery pack. The electric stapler is mainly used to perform two operation actions, such as jaw deflection and anastomotic cutting, for the target tissue. The jaw deflection action may be driven manually or by a motor, and the anastomotic cutting action is achieved by being driven by a motor.

For the main problems of electric staplers at present, the related art provides different solutions, which may avoid dealing with most situations, but there are also certain defects.
(1) The tissue squeezing of the semi-automatic staplers (manual closure, manual deflection, electric firing) and some fully automatic staplers without sensors are completely determined by the experience of the user (such as surgical doctors), and the training is provided before use, which is difficult to use and prone to problems. In addition, some fully automatic staplers are equipped with force sensors to provide force feedback to doctors, which may determine tissue hardness, toughness, etc. based on the feedback. However, additional force sensors are required, which incurs high design and manufacturing costs. As a disposable device, it brings significant economic pressure to patients, and the squeezing situation still needs to be determined by doctors. In addition, some fully automatic staplers use motor encoders to determine the squeezing position. However, the motor is used as the starting position of power, due to problems such as tolerances and clearances of the transmission mechanism, the value of the encoder may not accurately reflect the actual position of the squeezing travel.
(2) The existing electric staplers mostly use mechanical limiting methods for the problem of replacing the staple cartridge. A firing block is designed on the staple cartridge to open the stapler limit lock, allowing the firing function to be used. The firing block is a destructive structure that may be damaged after one firing, achieving the goal of the staple cartridge not being reused. However, the problem of this scheme is that during the firing process, the cutting knife uses a large firing force and a fast motor running speed, which may cause impact on the mechanical limit lock, causing deformation of the firing mechanism or a decrease in strength. There is a risk of structural damage in the next firing, and in severe cases, it may cause structural components to scatter, turning minimally invasive surgery into open surgery.

To solve at least one of the technical problems in the specific firing and other operations of the electric stapler in the related art, the present disclosure provides a travel detector and a method of controlling a travel detector, an electric stapler, and a medical device to add a detection device in high-precision medical device such as an electric stapler, which may determine the various operation stages of the specific firing and other operations.

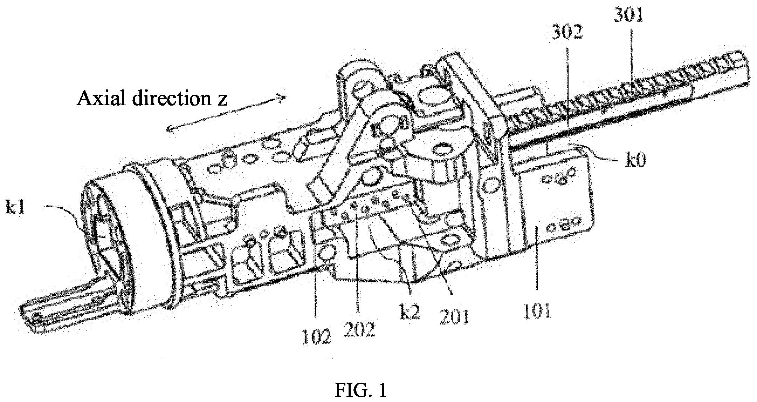

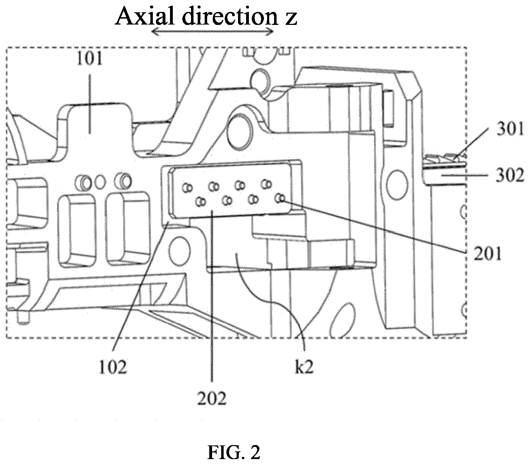

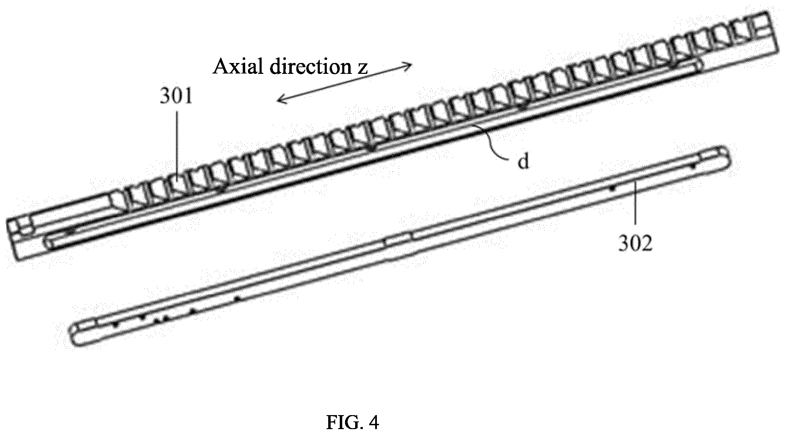

As shown in FIGS. 1 to 7, an aspect of the present disclosure provides a travel detector applied to a detection on a firing action of a medical device, including a supporting portion, a transmission portion, and a detecting portion.

The supporting portion serves as a supporting structure of the travel detector.

The transmission portion penetrates into the supporting portion and is arranged to be movable in an axial direction z in a supporting body.

The detecting portion is fixed on the supporting portion and is in contact with the transmission portion through the supporting portion.

An end of the transmission portion is connected to an operating end of the medical device. When the transmission portion moves in the axial direction z in the supporting portion, the transmission portion sequentially comes into contact with different detecting positions on the detecting portion to complete a travel detection of the transmission portion relative to the supporting portion, so that the operating end performs a firing action according to the travel detection.

The supporting portion is a main component portion of the travel detector, and is used to provide a main support for the travel detector, which may provide an internal space to accommodate other components or structures such as the transmission portion.

The transmission portion is provided on the supporting portion and may perform a movement action of entering or exiting relative to the supporting portion in the axial direction z. When the transmission portion enters the supporting portion, the transmission portion may drive the operating end of the medical device to perform a first action. When the transmission portion exits the supporting portion, the transmission portion may drive the operating end to perform a second action. The first action and the second action may be opposite actions such as opening and closing, or opposite actions such as left and right, or forward and backward, and specifically, such as feeding knife or retracting knife, forward deflection or reverse deflection, etc.

The detecting portion includes different detecting positions. By contacting the transmission portion through the different detecting positions, the detecting portion detects and feeds back the travel of the transmission portion entering or exiting the supporting portion by using the different detecting positions. When the transmission portion is in contact with different detecting positions, different electrical signals may be generated, and a corresponding electrical signal may represent a corresponding travel position of the transmission portion.

Therefore, by detecting the travel of the transmission portion, the problem of excessive squeezing may be completely avoided, and the detections for different detecting positions, such as a detection with the staple cartridge secondary firing lock, may be achieved, so as to effectively prevent the damage caused by the strong impact of the firing structure on the staple cartridge secondary firing lock. The entire structure is simple, the volume is compact, and the manufacturing cost is low, while ensuring high detection accuracy, and it overcame the technical problem of difficult integration of more detectors in the narrow mechanical space of handheld precision medical device such as electric staplers, and successfully disposing the detecting portion in the supporting portion, forming the only mechanical device in medical devices that may achieve high-precision travel detection of the transmission portion. The detection accuracy is high, the structure is simple, the volume is small, and the manufacturing cost is low.

As shown in FIGS. 1 to 7, according to embodiments of the present disclosure, the supporting portion includes a supporting frame 101 and a fixed plate 102.

The supporting frame 101 is a frame structure with a central space k0, and is provided with a plurality of different disposing positions for disposing the transmission portion and the detecting portion, respectively.

The fixed plate 102 corresponds to one of the plurality of different disposing positions, and is recessed on a side of the supporting frame 101 to form a disposing space k2 for the detecting portion. The fixed plate 102 includes a plurality of first fixed holes for fixing the detecting portion.

The fixed plate 102 is integrally formed with the supporting frame 101.

As a main body of a handle portion in the device, the frame structure may provide more disposing space for the transmission portion and the detecting portion through the central space k0. The central space k0 may be used to increase the disposing positions of the frame structure, which is conducive to further structural integration of the travel detector. The disposing position is used as a position for disposing a component on the frame structure, which may be a convex or concave structure or space, and in a plurality of regular or irregular shapes, without specific restrictions. It should be noted that, as shown in FIG. 1, an end face of the frame structure facing the operating end has a transmission hole k1. The transmission hole may be used for the linear transmission of the transmission portion in the axial direction z from the central space k0 to the transmission hole k1 when the transmission portion enters the supporting portion. Conversely, when the transmission portion exits the supporting portion, the transmission portion may perform a linear transmission in the axial direction z from the transmission hole k1 to the central space k0.

The fixed plate 102 is used to separate the transmission portion and the main structure of the detecting portion, so that the transmission portion may achieve contact detection with different contact positions of the detecting portion during the transmission process through the fixed plate 102. The disposing space k2 is a disposing position recessed on a side of the frame structure. The disposing space k2 is recessed towards the central space k0 with the outer surface of the fixed plate 102 as the bottom surface. A plurality of through holes are arranged on the fixed plate 102 according to the disposing rules, that is first fixed holes. The plurality of first fixed holes may be used to fix the detecting portion on the supporting frame 101 of the supporting portion in conjunction with the disposing space k2. And, a contact between the detecting portion and the transmission portion in the central space k0 may also be achieved through the plurality of first fixed holes. The fixed plate 102 may limit and fix the detecting portion with the help of the first fixed hole and the disposing space k2.

As shown in FIGS. 1 to 7, according to embodiments of the present disclosure, the detecting portion includes a plurality of thimbles 201 and a thimble plate 202.

The plurality of thimbles 201 correspond to the plurality of first fixed holes one by one. A hard sheath 211 of each of the plurality of thimbles 201 penetrates through a corresponding first fixed hole. A head 212 of the thimble 201 is facing towards an inside of the supporting frame 101.

The thimble plate 202 is matched with the fixed plate 102, and is disposed in the disposing space k2.

A limiting seat 213 of each thimble 201 is disposed between the thimble plate 202 and the fixed plate 102.

The thimble 201 includes a hard sheath 211, a head 212, a limiting seat 213, and a fixed head 214. The head 212 is pressed into the hard sheath 211 to maintain a specific elastic force, and protrudes outward relative to the hard sheath 211 under the elastic force of the spring in the hard sheath 211. The top of the head 212 may be an end of a hemisphere to facilitate to be in contact with the transmission portion. In addition, as a supporting and limiting structure, the limiting seat 213 may be integrally formed or fixedly connected to the hard sheath 211 and the fixed head 214. A diameter of the limiting seat 213 is greater than a diameter of the hard sheath 211. The diameter of the hard sheath 211 is greater than a diameter of the head 212 or a diameter of the fixed head 214. In practice, a length of the hard sheath 211 is 4.2mm, and a length of the head 212 is 1.5mm. In order to maintain stable contact, the head 212 is pre-pressed by 0.9mm, leaving a certain range of fluctuations for processing and assembly errors. The diameter of the limiting seat 213 is about 2.2mm, which forms the diameter size of the thimble 201.

In addition, the length of the hard sheath 211 may be identical to the thickness of the fixed plate 102, so as to ensure that the head may be exposed towards the central space k relative to the fixed plate 102, thereby achieving contact between the head 212 of the thimble 201 and the transmission portion, and also preventing the thimble 201 from inclining in the first fixed hole. Moreover, the diameter of the hard sheath 211 is set to be matched with the size of the first fixed hole, so that the thimble 201 will not shake when penetrating the first fixed hole during the process of the travel detection, ensuring the accuracy and stability of the detection process.

The thimble plate 202 is used as a fixed structure of the thimble 201. The actual size of the thimble plate 202 may be set to 20mm*5mm or 21 mm*5.5mm. The thimble plate 202 is matched with a fixed board 102 and installed at the position of the disposing space k2. Specifically, the hard sheath 211 of each thimble 201 is inserted into the first fixed hole of the fixed plate 102, and the head 212 is disposed towards the inside of the supporting frame 101.

It should be noted that the thimble plate 202 is provided with a thimble circuit board, which may be used to process and convert the contact electrical signals received by the thimble, forming detection data of different detecting positions.

As shown in FIG. 1 to FIG. 7, according to embodiments of the present disclosure, the thimble plate 202 includes a plurality of second fixed holes g1. The plurality of thimbles 201 correspond to the plurality of second fixed holes g1 one by one. A fixed head 214 of each thimble 201 of the plurality of thimbles 201 penetrates through a corresponding second fixed hole g1. The fixed head 214 is facing towards an outside of the supporting frame 101.

The fixed head 214 is correspondingly inserted into the second fixed hole g1, so that there may be only a limiting seat 213 as an interval between the fixed plate 102 and the thimble plate 202, thereby completing the fixation of the thimble plate 202 with the fixed plate 102 through the thimble 201. At this point, the fixed head 214 is facing towards the outer side of the disposing space k2.

As shown in FIG. 1 to FIG. 7, according to embodiments of the present disclosure, the plurality of thimbles 201 include a first set of thimbles and a second set of thimbles.

The first set of thimbles is disposed corresponding to a set of first fixed holes on the fixed plate 102.

The second set of thimbles is disposed corresponding to a set of second fixed holes on the fixed plate 102. A setting spacing is between the second set of thimbles and the first set of thimbles.

The plurality of thimbles 201 may be mainly divided into two sets. The first set of thimbles is disposed above the second set of thimbles with the setting spacing, and the number of thimbles 201 in each set may be the same. The number of thimbles 201 is identical to the number of second fixed holes or the number of first fixed holes. For example, two sets of second fixed holes are opened on the thimble plate 202, corresponding to the first set of thimbles and the second set of thimbles, respectively. The two sets of thimbles formed by the plurality of thimbles 201 cooperate with the thimble plate 202, which are arranged in upper and lower rows.

As shown in FIG. 1 to FIG. 7, according to embodiments of the present disclosure, the first set of thimbles and the second set of thimbles are staggered in the axial direction. A spacing between any two adjacent thimbles 201 in the axial direction is equal. The first set of thimbles and the second set of thimbles each includes at least one thimble 201 connected to a corresponding detecting common end.

For further saving space and facilitating to dispose a detecting portion in a limited space, a plurality of second fixed holes may be staggered arranged on the thimble plate 202 corresponding to the fixed plate 102 to dispose the first set of thimbles and the second set of thimbles, so that the thimbles are staggered arranged in the axial direction z. In each set of thimbles, a spacing between two adjacent thimbles 201 in the axial direction z is 4mm, and a spacing between centers of two adjacent thimbles 201 arranged side by side from top to bottom is 1.6mm, so that the thimble plate 202 may accommodate more thimbles arranged side by side.

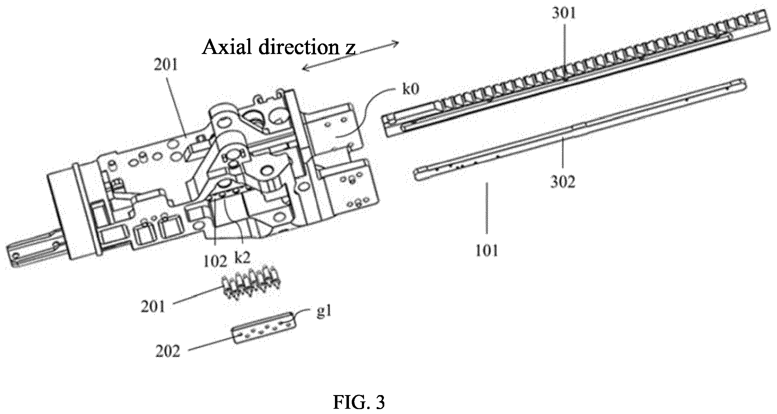

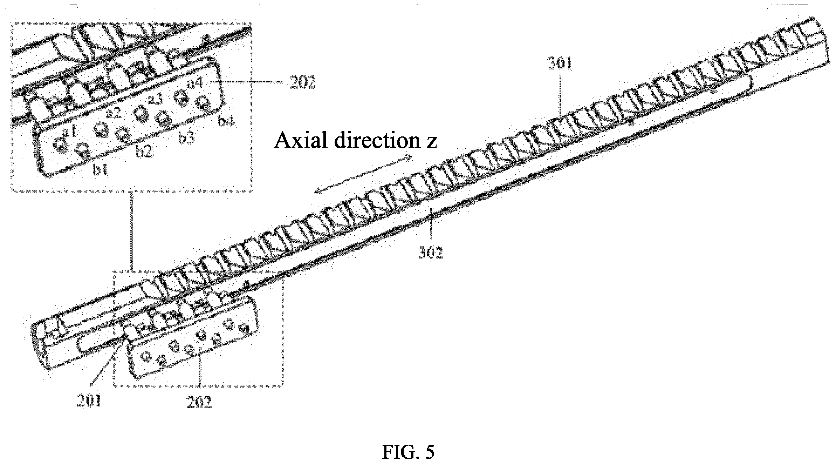

As shown in FIG. 3 and FIG. 5, the thimble plate 202 may be provided with 8 thimbles 201, which are staggered arranged in 2 rows. The upper row is the first set of thimbles, and the lower row is the second set of thimbles. Each set of thimbles has 4 thimbles 201 which are staggered arranged and satisfy as follows.

The first set of thimbles (upper row) includes 1 to 4 thimbles (left to right): a1, a2, a3, and a4. During the movement of the transmission portion, the thimbles a1, a2, a3, and a4 are electrically connected to the electrical contacts of L', Z', COM1, and COM2 on the circuit board of the thimble plate 202 through respective fixed heads 214 of the thimbles a1, a2, a3, so as to respectively achieve a secondary firing lock position detection and a retraction zero position detection.

The second set of thimbles (lower row) includes 1 to 4 thimbles (left to right): b1, b2, b3, and b4. During the movement of the transmission portion, the thimbles b1, b2, b3, and b4 are electrically connected to the electrical contacts of F', C', COM3, and COM4 on the circuit board of the thimble plate 202 through respective fixed heads 214 of the thimbles b1, b2, b3, and b4, so as to respectively achieve a travel endpoint position detection and a closed zero position detection. As any contact of L', Z', F', and C' may form an independent detecting loop with the COM terminal (one of COM1 to COM4), a valid detection signal may be output.

As shown in FIG. 1 to FIG. 7, according to embodiments of the present disclosure, the transmission portion includes a transmission strip 301 and a circuit board 302.

The transmission strip 301 is a long rod-shaped structure. An end of the transmission strip is connected to the operating end. When the transmission strip 301 moves relative to the central space k0 of the supporting frame 101 in the axial direction z, the transmission strip transmits an action of a forward and backward movement to the operating end.

The circuit board 302 is a long plate-shaped structure disposed on a side surface of the transmission strip 301. When the transmission strip 301 moves relatively in the axial direction z, the circuit board 302 comes into different contacts with the plurality of thimbles 201 to determine different travels of the transmission strip 301, so that the operating end completes the travel detection of the firing action.

The transmission strip 301 may be a long rod-shaped rack structure. Continuous toothed protrusions are provided on a side surface of the transmission strip, and are used to drive the transmission strip 301 to move relative to the supporting portion in the axial direction. A long strip-shaped groove d is provided on the surface of a side of the transmission strip 301 facing towards the fixed plate 102 and the thimble plate 202. The groove d is used to accommodate the circuit board 302.

The circuit board 302 is a long strip-shaped structure matched with the groove d of the transmission strip 301, with a certain thickness. A plurality of different detecting positions are disposed on the surface of the circuit board 302 facing the fixed plate 102 and the thimble plate 202, and are used to achieve the detection of the position of the transmission strip by the detecting portion, during the process of the transmission strip entering or exiting in the axial direction.

An actual size of circuit board 302 may be 100mm * 3mm. An adhesive material such as safety glue is used to stick the circuit board in the groove d of the transmission strip 301. The circuit board 302 may also be limited in the groove d by the convex structure at the middle or two ends of circuit board 302 in a length direction. The structural design of the circuit board 302 is very precise and small, which poses certain objective difficulties in design and manufacturing. Moreover, in practical applications, it is not a conventional ordinary structural design.

The detecting position is used to identify the travel position of the transmission strip 301, which may be a contact of the electrical contact. By relative movement between the plurality of thimbles 201 on the thimble plate 202 and the circuit board 302 on the transmission strip 301, the contact of the thimble 201 at different positions on the circuit board 302 is achieved, completing the travel detection.

Therefore, the structural design of the above detecting portion is extremely simple, compact in size, and has little impact on the main structure of the medical device, while the manufacturing cost is low, and the detection function may cover all operational actions of the firing action of the medical device such as staplers.

As shown in FIG. 1 to FIG. 7, according to embodiments of the present disclosure, the circuit board includes a plurality of contacts. The plurality of contacts include a first set of contacts and a second set of contacts.

The first set of contacts is in corresponding contact with the first set of thimbles, and is used to achieve a retraction zero position detection and a secondary firing lock position detection.

The second set of contacts is in corresponding contact with the second set of thimbles, and is used to achieve a closed zero position detection and a travel endpoint position detection.

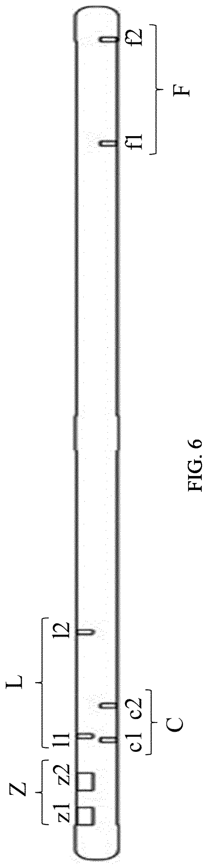

To ensure that the conventional machining size of the pad on circuit board 302 and the size of the contact surface of the thimble may effectively distinguish two adjacent detecting contacts, the pad is designed as two rows of an upper row and a lower row. Therefore, the plurality of contacts form a first set of contacts in corresponding contact with the plurality of thimbles of the first set of thimbles and a second set of contacts in corresponding contact with the plurality of thimbles of the second set of thimbles, which are respectively located in the upper row and the lower row of the thimble plate 202. As shown in FIG. 6, the first set of contacts (upper row) includes 1 to 4 contacts (left to right): z1, z2, 11, and I2, which are respectively in contact with some thimbles among the thimbles of the first set of thimbles a1, a2, a3, and a4 during the movement of the transmission portion. During different contact processes, the retraction zero position detection and the secondary firing lock position detection are achieved. The second set of contacts (lower row) includes 1 to 4 contacts (left to right): c1, c2, f1, and f2, which are respectively in contact with some thimbles among the thimbles of the first set of thimbles b1, b2, b3, and b4 during the movement of the transmission portion. During different contact processes, the closed zero position detection and the travel endpoint position detection are achieved. The linear spacing between the first set of contacts in the upper row and the second set of contacts in the lower row is only 0.4mm, which is extremely precise in design.

As shown in FIG. 1 to FIG. 7, according to embodiments of the present disclosure, the first set of contacts includes a first sub-set of contacts Z and a second sub-set of contacts L.

The first sub-set of contacts Z includes two contacts provided at a top of the circuit board 302. The two contacts are respectively in contact with two adjacent thimbles 201 in the first set of thimbles to achieve the retraction zero position detection.

The second sub-set of contacts L includes two contacts provided adjacent to the first sub-set of contacts Z. The two contacts are respectively in contact with first and last thimbles in the first set of thimbles to achieve the secondary firing lock position detection.

The transmission strip 301 of the transmission portion moves forward and backward in a straight line in the axial direction z at the starting position, that is, the anvil is retracted to the zero position. The corresponding pad design of circuit board 302 at this point is 2mm. Pad contacts on the circuit board 302 are connected in pairs to output different electrical signals.

As shown in FIG. 6, the first sub-set of contacts Z includes two contacts z1 and z2. The two contacts z1 and z2 are respectively used to contact the two thimbles a2 and a3 in the first set of thimbles during the transmission process of the transmission strip 301, so as to conduct the electrical contacts Z' and COM1 of the thimble plate 202 and output a Z signal. The Z signal is used for the retraction zero position detection of the anvil. The corresponding pad spacing on the circuit board 302 is 4mm, and the pad size is 2mm * 1.2mm.

The second sub-set of contacts L includes two contacts l1 and l2, which are respectively used to contact the two thimbles a1 and a4 in the first set of thimbles during the transmission process of the transmission strip 301, so as to conduct the electrical contacts L' and COM2 of the thimble plate 202 and output an L signal. The L signal is used for the secondary firing lock position detection of the staple cartridge. The corresponding pad spacing on the circuit board 302 is 12mm, and the pad size is 0.5mm * 1.2mm.

As shown in FIG. 1 to FIG. 7, according to embodiments of the present disclosure, the second set of contacts includes a third sub-set of contacts C and a fourth sub-set of contacts F.

The third sub-set of contacts C includes two contacts located on a lower side of the first sub-set of contacts Z. The two contacts are respectively in contact with two adjacent thimbles in the second set of thimbles to achieve the closed zero position detection.

The fourth sub-set of contacts F includes two contacts provided at an end of the circuit board 302. The two contacts are respectively in contact with first and last thimbles 201 in the second set of thimbles to achieve the travel endpoint position detection.

As shown in FIG. 6, the third sub-set of contacts C includes two contacts c1 and c2. The two contacts c1 and c2 are respectively used to contact the two thimbles b2 and b3 in the second set of thimbles during the transmission process of the transmission strip 301, so as to conduct the electrical contacts C' and COM3 of the thimble plate 202 and output a C signal. The C signal is used for closed zero position detection of the anvil. The corresponding pad spacing on the circuit board 302 is 4mm, and the pad size is 0.5mm * 1.2mm.

The second sub-set of contacts F includes two contacts f1 and f2, which are respectively used to contact the two thimbles b1 and b4 in the second set of thimbles during the transmission process of the transmission strip 301, so as to conduct the electrical contacts F' and COM4 of the thimble plate 202 and output an F signal. The F signal is used for the endpoint position detection of the cutting knife. The corresponding pad spacing on the circuit board 302 is 12mm, and the pad size is 0.5mm * 1.2mm.

Therefore, through the extremely simple contact structure distribution design described above, the circuit board 302 of the transmission portion may be matched with the plurality of thimbles 201 of the detecting portion to achieve accurate detection of the linear transmission distance in the axial direction z of the transmission portion. The entire structure is safe, stable, and reliable. By adding position detection, it may effectively prevent accidental damage to the tissue and the risk of expanding the surgical range caused by clamping too thick tissue. In addition, it may effectively avoid the impact of firing locks on the structure and reduce the hidden dangers of device use. Moreover, it is possible to further increase the testing points at each stage of the travel, so that medical devices such as electric staplers operated in the body may change the output cutting torque or control method through different travel positions of the firing, providing a more detailed and accurate surgical process.

It should be noted that the design structure of the coordination between the thimble and the contact shown in FIG. 1 to FIG. 7 above is only for explaining embodiments of the technical solution of the present disclosure, and is not a specific limitation of the protection scope of the present disclosure. For example, the number of thimbles in the set of thimbles is not limited to 8, and the number of contacts in the corresponding set of contacts is not limited to 8. Specifically, in the coordination design of the thimbles a3 and a4 in the first set of thimbles and the connected COM1 and COM2, the pair of COM1 and a3 or the pair of COM2 and a4 may be removed. Correspondingly, in the coordination design of the thimbles b3 and b4 in the second set of thimbles and the connected COM3 and COM4, the pair of COM3 and b3 may be removed or the pair of COM4 and b4 may be removed, so that only 6 thimbles is retained in the set of thimbles, while adjusting the distribution of their corresponding contact circuits and contacts. The above-described travel detection technical solution may still be achieved. It is also possible to further increase the corresponding number of contacts and thimbles, etc., which will not be repeated here.

As shown in FIG. 1 to FIG. 7, according to embodiments of the present disclosure, a first spacing between the two contacts in the first sub-set of contacts Z in the axial direction is equal to a third spacing between the two contacts in the third sub-set of contacts C in the axial direction. A second spacing between the two contacts in the second sub-set of contacts L in the axial direction is equal to a fourth spacing between the two contacts in the fourth sub-set of contacts F in the axial direction.

The width of the two contacts in the first sub-set of contacts Z in the axial direction is greater than the width of the two contacts in the second sub-set of contacts L in the axial direction, which allows to perform the retraction zero position detection of the anvil at the beginning of the detection to ensure the detection margin and prevent the contacts z1 and z2 from being not effectively released from the corresponding thimbles a2 and a3, which affects the progress of the detection process. The width of the two contacts in the second sub-set of contacts L in the axial direction is equal to the width of the two contacts in the third sub-set of contacts C in the axial direction. The width of the two contacts in the second sub-set of contacts L in the axial direction is equal to the width of the two contacts in the fourth sub-set of contacts F in the axial direction.

The eight different detection contacts z1-f2 on the circuit board 302 may be designed with different spacing sizes, so as to ensure that the same detecting point does not trigger the plurality of sets of thimbles during the processes of the knife entering and exiting. The spacing between the detecting contacts of the first sub-set of contacts Z and the second sub-set of contacts L is 5.25mm. The spacing between the detecting contacts of the third sub-set of contacts C and the fourth sub-set of contacts F is 64.8mm, which conforms to the structural dimensions of the firing process. Moreover, the first sub-set of contacts Z and the second sub-set of contacts L are detected on the upper side of the circuit board 302, while the third sub-set of contacts C and the fourth sub-set of contacts F are detected on the lower side of circuit board 302. The linear spacing between the sets of contacts used for the upper and lower detections is 0.4mm, and the spacing between each set of contacts and the board edge is 0.2mm. Therefore, the entire width of the circuit board 302 from top to bottom is 3mm, which is similar to the current limit size of conventional PCB processing. There is no need to increase the cost of designing the processing method for the limit circuit board 302. Due to being divided into upper and lower rows, the detection requirements for two points closer to each other are reduced. The contact portion of the thimble tip may also use conventional design, resulting in a higher yield.

Therefore, as shown in FIG. 1 to FIG.7, based on embodiments of the travel detector of the present disclosure, the operating end being a front end jaw component of the electric stapler is taken as an example, the specific process of travel position detection may be further summarized as follows.

When the jaw of the front jaw component is fully opened, the transmission strip 301 is in original position (i.e. the retraction zero position of the anvil). At this time, the zero position detection contacts z1 and z2 on the circuit board 302 are conducted with the retraction zero position detection contacts Z' 'and COM1 on the entering/exiting knife thimble plate 202 through the thimbles a2 and a3, completing the retraction zero position detection.

Then, the motor drives the transmission strip 301 to move forward in a straight line in the axial direction z, driving the circuit board 302 to move. When the anvil is completely closed and meets the anastomosis cutting conditions, the larger spaced contacts c1 and c2 on the circuit board 302 are conducted with the closed zero position detection contacts C' and COM3 on the thimble plate 202 through the thimbles b2 and b3, completing the closed zero position detection of the anvil and achieving precise control of the closed squeezing of the anvil.

Correspondingly, the transmission strip 301 continues to move forward and completes the secondary firing lock detection of the staple cartridge and the endpoint position detection of the cutting knife at different spaces and positions sequentially, which will not repeated here.

Therefore, by detecting the travel of the transmission portion, the problem of excessive squeezing may be completely avoided, and the detection for different detecting positions, such as a detection with the staple cartridge secondary firing lock, may be achieved, so as to effectively prevent the problem of damage caused by the strong impact of the firing structure on the staple cartridge secondary firing lock. The entire structure is simple, the volume is compact, and the manufacturing cost is low, while ensuring high detection accuracy. And, the technical problem of difficult integration of more detectors in the narrow mechanical space of handheld precision medical device such as electric staplers is also overcome, thereby successfully disposing the detecting portion in the supporting portion, forming the only mechanical device in medical devices that may achieve high-precision travel detection of the transmission portion. The detection accuracy is high, the structure is simple, the volume is small, and the manufacturing cost is low.

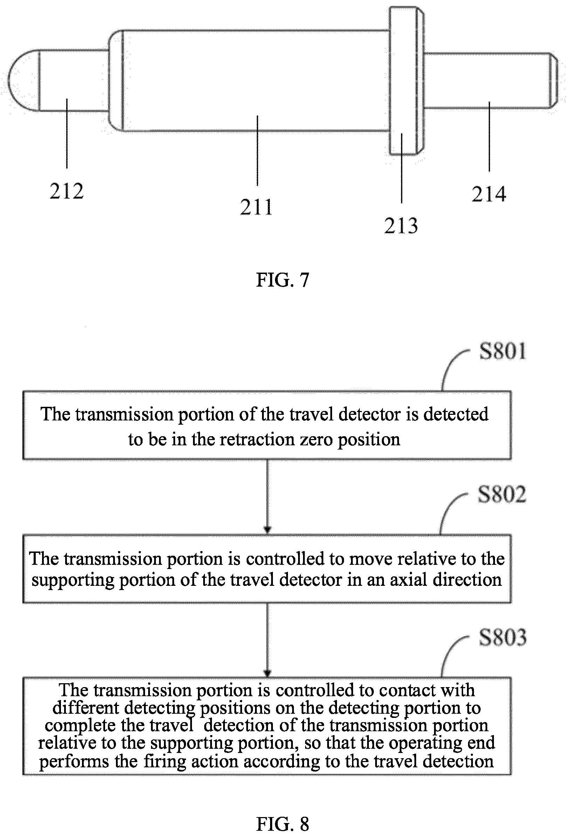

As shown in FIG. 7, another aspect of the present disclosure provides a method of controlling the travel detector described above, applied to a detection on a firing action of a medical device, including steps S801 to S803.

In step S801, the transmission portion of the travel detector is detected to be in the retraction zero position, in response to a detection instruction.

In step S802, the transmission portion is controlled to move relative to the supporting portion of the travel detector in an axial direction, in response to the retraction zero position.

In step S803, the transmission portion is controlled to contact with different detecting positions on the detecting portion to complete the travel detection of the transmission portion relative to the supporting portion, so that the operating end performs the firing action according to the travel detection.

The detection instruction may be a detection electrical signal generated by the user (such as a surgeon) manually triggering the switch button, and be used to indicate the start of the travel detection process.

For achieving the control method shown in FIG. 8, embodiments of the present disclosure provides a measurement and control system for implementing the control method.

Firstly, the new electric stapler and the jaw component provided in embodiments of the present disclosure are taken as an example, the method of controlling the travel detection of the electric stapler is further described in detail as follows.

First firing process: the closing process of the anvil and the seat of the staple cartridge is optimized. The installing of the staple cartridge is determined by electronic switches, and the closing squeezing and anastomosis cutting actions are finely distinguished to prevent the firing mechanism from exerting excessive torque on the mechanical limiting structure.

The staple cartridge detection: the replacement of the staple cartridge and the installing correct detection method are optimized. In addition to the mechanical limiting, an electronic secondary firing lock is added to prevent the impact of excessive motor force on the mechanical structure.

Secondary firing process: the anastomosis, cutting and completion of the retraction process after the staple cartridge is detected, and the anastomosis and cutting actions are performed. After completion, the mechanism electrically retracts and automatically opens the anvil and the seat of the staple cartridge, and the closed-loop control algorithm is used, with action prompts and error indications.

In combination with the content shown in FIG. 1 to FIG. 7, for the firing process, the new electric stapler subdivides the firing process into multiple processes: a first firing, a secondary firing and a retraction. The specific explanation is as follows.

The first firing includes the closing operation of the anvil and the seat of the stable cartridge, that is, the process of squeezing the tissue.

As shown in FIG. 1, the circuit board 302 may enable the detecting portion to detect four travel positions of the transmission portion, namely the anvil retraction zero position detection, the anvil closed zero position detection, the cutting knife secondary firing lock position detection, and the cutting knife endpoint position detection.

Specifically, when the entering knife button is pressed, the entering knife button transmits the electrical signal of the detection instruction to the main board. The mainboard controls the rotation of the firing motor. The rotation of the firing motor drives the motor gear. The motor gear drives the firing rod. The firing rod pushes the cutting knife forward. The cutting knife pushes the anvil along the inclined surface. When the anvil reaches the closing condition, the circuit board 302 is triggered. The circuit board 302 outputs a signal to the main board. The main board stops the rotation of the firing motor and causes the left and right safety buttons to flash, prompting the user to perform a secondary firing operation. The user must press the left and right safety buttons firstly, and the next actions of anastomosis and cutting may be performed, thus completing the first firing process.

After the first firing operation is completed, the secondary firing operation is continued performed. First, the secondary firing lock detection is performed to determine whether the staple cartridge is installed properly. The secondary firing lock is used to limit the cutting knife and the firing rod, preventing the cutting knife from making incorrect cutting actions when the staple cartridge is not installed.

The staple cartridge is installed on the seat of the staple cartridge. The push staple slider on the staple cartridge pushes the secondary firing lock and the mirror secondary firing lock to the right. If there is a problem with the installation of the staple cartridge or it is not installed, the secondary firing lock and the mirror secondary firing lock is closed under the action of the secondary firing lock return spring, blocking the forward movement of the cutting knife.

When the secondary firing action is started, the firing rod pushes the cutting knife forward, and the cutting knife pushes the push staple slider forward. During the movement, the driving force current is limited to 2A, and the low current torque effectively protects the cutting knife and the firing rod structure.

After the cutting knife is pushed forward through the secondary firing lock position, the system adjusts the driving force current limit to use high current torque for the anastomosis and cutting actions of the tissue.

The main board of the electric stapler automatically adjusts the firing torque based on the position of the cutting knife. The main process region may be divided into low torque firing process before the anastomosis and cutting, high torque anastomosis cutting and retraction process. The anastomosis, cutting and retraction processes start from the cutting knife reaching the secondary firing lock position.

Another aspect of the present disclosure provides an electric stapler, including the travel detector as described above and a jaw component.

The above travel detector is used to complete the travel detection of the transmission portion relative to the supporting portion.

The jaw component is connected to the supporting portion of the travel detector and serves as an operating end to perform a firing action according to the travel detection.

Specifically, the travel detector and the method of controlling the travel detector in embodiments of the present disclosure may be referred to FIGS. 1 to 8 above, which will not be repeated here.

Another aspect of the present disclosure provides a medical device, including the travel detector as described above.

It may be seen that the medical device in embodiments of the present disclosure may include a new type of electric endoscope linear cutting stapler for dissection, resection, and/or establishment of anastomosis, which is mainly suitable for operations of open or minimally invasive general surgery, obstetrics and gynecology, urology, thoracic surgery, and pediatric surgery, and may be in cooperation with anastomosis lines or tissue support materials, and may also be used for dissection and resection of liver parenchymal tissue (liver vascular system and biliary structure), pancreas, kidney, and spleen. The electric endoscope linear cutting stapler includes the body and component parts, and is a disposable product that may only be used in one surgery. Due to the disposable nature of the electric endoscope linear cutting stapler, cross infection during surgery is avoided.

At this point, a detailed description of embodiments of the present disclosure has been provided in conjunction with the accompanying drawings.

The specific embodiments described above further elaborate on the purposes, technical solutions, and beneficial effects of the present disclosure. It should be understood that the above are only specific embodiments of the present disclosure and are not intended to limit the present disclosure. Any modifications, equivalent substitutions, improvements, etc. made within the spirit and principles of the present disclosure should be included in the scope of protection of the present disclosure.

## Claims

1. A travel detector, applied to a detection on a firing action of a medical device, comprising:
a supporting portion serving as a supporting structure of the travel detector;
a transmission portion penetrating into the supporting portion and arranged to be movable in an axial direction in a supporting body; and
a detecting portion fixed on the supporting portion and in contact with the transmission portion through the supporting portion,
wherein an end of the transmission portion is connected to an operating end of the medical device, and when the transmission portion moves in the axial direction in the supporting portion, the transmission portion sequentially comes into contact with different detecting positions on the detecting portion to complete a travel detection of the transmission portion relative to the supporting portion, so that the operating end performs the firing action according to the travel detection.

2. The travel detector of claim 1, wherein the supporting portion comprises:
a supporting frame being a frame structure with a central space, and provided with a plurality of different disposing positions for disposing the transmission portion and the detecting portion respectively; and
a fixed plate corresponding to one of the plurality of different disposing positions, and recessed on a side of the supporting frame to form a disposing space for the detecting portion, wherein the fixed plate comprises a plurality of first fixed holes for fixing the detecting portion,
wherein the fixed plate is integrally formed with the supporting frame.

3. The travel detector of claim 2, wherein the detecting portion comprises:
a plurality of thimbles corresponding to the plurality of first fixed holes one by one, wherein a hard sheath of each of the plurality of thimbles penetrates through a corresponding first fixed hole, and a head of the thimble is facing towards an inside of the supporting frame; and
a thimble plate matched with the fixed plate and disposed in the disposing space,
wherein a limiting seat of each of the plurality of thimbles is disposed between the thimble plate and the fixed plate.

4. The travel detector of claim 3, wherein the thimble plate comprises:
a plurality of second fixed holes corresponding to the plurality of thimbles one by one, wherein a fixed head of each of the plurality of thimbles penetrates through a corresponding second fixed hole, and the fixed head is facing towards an outside of the supporting frame.

5. The travel detector of claim 3, wherein the plurality of thimbles comprise:
a first set of thimbles disposed corresponding to a set of first fixed holes on the fixed plate; and
a second set of thimbles disposed corresponding to a set of second fixed holes on the fixed plate, wherein a setting spacing is between the second set of thimbles and the first set of thimbles.

6. The travel detector of claim 5, wherein the first set of thimbles and the second set of thimbles are staggered in the axial direction;
a spacing between any two adjacent thimbles in the axial direction is equal; and
the first set of thimbles and the second set of thimbles each comprises at least one thimble connected to a corresponding detecting common end.

7. The travel detector of claim 6, wherein the transmission portion comprises:
a transmission strip being a long rod-shaped structure, wherein an end of the transmission strip is connected to the operating end, and when the transmission strip moves relative to the central space of the supporting frame in the axial direction, the transmission strip transmits an action of a forward and backward movement to the operating end; and
a circuit board being a long plate-shaped structure disposed on a side surface of the transmission strip, wherein when the transmission strip moves relatively in the axial direction, the circuit board comes into different contacts with the plurality of thimbles to determine different travels of the transmission strip, so that the operating end completes the travel detection of the firing action.

8. The travel detector of claim 7, wherein the circuit board comprises a plurality of contacts, and the plurality of contacts comprise:
a first set of contacts, in corresponding contact with the first set of thimbles, and configured to achieve a retraction zero position detection and a secondary firing lock position detection; and
a second set of contacts, in corresponding contact with the second set of thimbles, and configured to achieve a closed zero position detection and a travel endpoint position detection.

9. The travel detector of claim 8, wherein the first set of contacts comprises:
a first sub-set of contacts comprising two contacts provided at a top of the circuit board, wherein the two contacts are respectively in contact with two adjacent thimbles in the first set of thimbles to achieve the retraction zero position detection; and
a second sub-set of contacts comprising two contacts provided adjacent to the first sub-set of contacts, wherein the two contacts are respectively in contact with first and last thimbles in the first set of thimbles to achieve the secondary firing lock position detection.

10. The travel detector of claim 9, wherein the second set of contacts comprises:
a third sub-set of contacts comprising two contacts provided on a lower side of the first sub-set of contacts, wherein the two contacts are respectively in contact with two adjacent thimbles in the second set of thimbles to achieve the closed zero position detection; and
a fourth sub-set of contacts comprising two contacts provided at an end of the circuit board, wherein the two contacts are respectively in contact with first and last thimbles in the second set of thimbles to achieve the travel endpoint position detection.

11. The travel detector of claim 10, wherein a first spacing between the two contacts in the first sub-set of contacts in the axial direction is equal to a third spacing between the two contacts in the third sub-set of contacts in the axial direction; and
a second spacing between the two contacts in the second sub-set of contacts in the axial direction is equal to a fourth spacing between the two contacts in the fourth sub-set of contacts in the axial direction,
wherein a width of the two contacts in the first sub-set of contacts in the axial direction is greater than a width of the two contacts in the second sub-set of contacts in the axial direction, the width of the two contacts in the second sub-set of contacts in the axial direction is equal to a width of the two contacts in the third sub-set of contacts in the axial direction, and the width of the two contacts in the second sub-set of contacts in the axial direction is equal to a width of the two contacts in the fourth sub-set of contacts in the axial direction.

12. A method of controlling the travel detector of any one of claims 1 to 11, applied to a detection on a firing action of a medical device, comprising:
detecting that the transmission portion of the travel detector is located at the retraction zero position, in response to a detection instruction;
controlling the transmission portion to move relative to the supporting portion of the travel detector in the axial direction, in response to the retraction zero position; and
controlling the transmission portion to contact with different detecting positions on the detecting portion to complete the travel detection of the transmission portion relative to the supporting portion, so that the operating end performs the firing action according to the travel detection.

13. An electric stapler, comprising:
the travel detector of any one of claims 1 to 11, configured to detect the travel of the transmission portion of the travel detector relative to the supporting portion of the travel detector; and
a jaw component connected to the supporting portion of the travel detector and serving as the operating end to perform the firing action according to the travel detection.

14. A medical device, comprising the travel detector of any one of claims 1 to 11.
